# EUROPEAN PATENT APPLICATION

(11) **EP 4 474 806 A1**
(43) Date of publication of application: **11.12.2024**
(21) Application number: 23749842.3
(22) Date of filing: 02.02.2023
(51) Int. Cl.: G01N 27/00, C12M 1/34, G01N 15/12

(54) **PARTICLE MEASUREMENT METHOD AND PARTICLE MEASUREMENT SENSOR DEVICE USED FOR SAME**

(30) Priority: 02.02.2022 JP 2022015252
(71) Applicant: ASAHI FR R&D Co., Ltd., Saitama-shi, Saitama 330-0801 (JP); Aipore Inc., Tokyo, 150-8512 (JP)
(72) Inventor: SHIMAMURA, Kazuki, Saitama-shi, Saitama 330-0801 (JP); TAKANO, Tsutomu, Saitama-shi, Saitama 330-0801 (JP); TAKEI, Hiroyasu, Tokyo 150-8512 (JP); NAONO, Norihiko, Tokyo 150-8512 (JP)
(74) Representative: J A Kemp LLP
(86) International application number: PCT/JP2023/003476
(87) International publication number: WO 2023/149526

(57) **Abstract**

A particle measurement method uses a particle measurement sensor device 300 having a Si monocrystalline substrate 290 in which a Si monocrystalline communication portion 299 is recessed and a pointed tip opening 101 to the Si monocrystalline communication portion 299 is opened, wherein a thin film 200 covering the entire pointed tip opening 101 is in contact with the Si monocrystalline substrate 290, and the thin film 200 is provided with a pore 209 that is in communication with the pointed tip opening 101, and the particle measurement sensor device 300 comprises: a measuring particle injecting side flow path 210 and a measuring particle non-injection side flow path 220 that sandwich the Si monocrystalline substrate 290 and are in communication with the Si monocrystalline communication portion 299; and a measuring particle injection side flow path connecting portion 219 and a measuring particle non-injection side flow path connecting portion 229 to the Si monocrystalline communication portion 299, and wherein a hydrophilic agent does not adhere to the measuring particle injecting side flow path 220 and to the measuring particle injecting side connecting portion 229.

## Description

### [Technical Field]

The present invention relates to a particle measurement method for performing particle measurement, particle count, and/or particle identification based on electrical resistance porosity analysis, as well as a particle measurement sensor device used for the same.

### [Background Art]

Various methods are known for measuring submicron-sized identifying particles, such as viruses, bacteria, and exosomes in a specimen, including an optical method such as an induced diffraction grating method (Patent Literature 1) and laser doppler electrophoresis (Patent Literature 2), a method using nucleic acid probes (Patent Literature 3), and a method using antigen-antibody reaction (Patent Literature 4). However, these methods have problems of lower accuracy, many analysis procedures, and higher costs. In particular, when the identifying particles are bacteria or viruses, lower concentrations of the identifying particles in a specimen often require incubation of the specimen, and no immediate means for measurement has been provided.

Therefore, recently, as a means for measuring submicron-sized measuring particles, a pore electrical resistance method of measuring a time change of an ion current when the identifying particles in an electrolyte pass through a pore has been proposed (Patent Literatures 5 and 6). Further, a technique has been proposed that combines the time change of the ion current with information processing by machine learning to identify what is the identifying particles, with high accuracy (Patent Literature 7). Furthermore, a device for measuring the time change of ion current has been proposed, which has a flow path including: a substrate provided with a through hole that is a passage for moving a substance; and a coated layer including an atomic layer provided on a wall surface of the through hole, wherein the device has a structure in which a pore on a thin film provided on a Si monocrystalline substrate is in communication with an opposite side of the Si substrate via a communication portion provided on the Si monocrystalline substrate (Patent Literature 8).

In order to apply these existing particle measurement sensor devices to industrial products for measuring various physical properties of particles, or to inspection equipment products for viruses, bacteria, etc., it has been necessary to solve the problem of stability of operation. However, there have been problems due to the hydrophobicity of the Si monocrystalline substrate (pore chip) and the flow path, and due to the complicated flow path shape. Specifically, there have been problems that air bubbles have remained in the flow path, especially in the bent portions, connected portion and corners of the flow path, which would prevent the test liquid from being delivered, or the identifying particles have been clogged and caused blockages or pulsations, making it impossible to accurately and stably measure particles such as fine particles, viruses, bacteria, etc. As a result, industrial or clinical applications of such particle measurement sensor devices have been hindered.

### [Citation List]

### [Patent Literatures]

[PTL 1]
   Japanese Patent Application Publication No. 2010-249607 A
[PTL 2]
   Japanese Patent Application Publication No. 2016-200608 A
[PTL 3]
   Japanese Patent Application Publication No. 2015-107091 A
[PTL 4]
   Japanese Patent Application Publication No. 2017-156324 A
[PTL 5]
   Japanese Patent Application Publication No. 2014-521962 A
[PTL 6]
   WO 2013/137209 A1
[PTL 7]
   Japanese Patent Application Publication No. 2017-120257 A
[PTL 8]
   WO 2020/230219 A1

### [Summary of Invention]

### [Technical Problem]

The present invention has been made to solve the above problems. An object of the present invention is to provide a particle measurement method that can smoothly deliver a test liquid without leaving air bubbles in a flow path or Si monocrystalline substrate (pore chip), can accurately and stably measure particles such as microparticles, viruses, bacteria, etc., without causing clogging due to identifying particles in the test liquid, or causing any obstructing or pulsating flow, and can be applied industrially, clinically, and commercially, as well as a particle measurement sensor device used for the same.

### [Solution to Problem]

In one embodiment, a particle measurement method according to the present invention made to achieve the above object is a particle measurement method, the method comprising:
using a particle measurement sensor device having a Si monocrystalline substrate in which a Si monocrystalline communication portion is recessed and a pointed tip opening to the Si monocrystalline communication portion is opened, wherein a thin film covering the entire pointed tip opening is in contact with the Si monocrystalline substrate, and the thin film is provided with a pore that is in communication with the pointed tip opening, and the particle measurement sensor device comprises: a measuring particle injecting side flow path and a measuring particle non-injection side flow path that sandwich the Si monocrystalline substrate and are in communication with the Si monocrystalline communication portion; and a measuring particle injection side flow path connecting portion and a measuring particle non-injection side flow path connecting portion to the Si monocrystalline communication portion; and
filling the measuring particle injecting side flow path and the measuring particle injecting side flow path connecting portion with an electrolyte mixed with measuring particles, filling the Si monocrystalline communication portion, the measuring particle non-injecting side connecting portion and the measuring particle non-injecting side flow path with a measuring particle-free electrolyte, and bringing the measuring particle injecting side flow path and the measuring particle non-injecting side flow path to a state capable of electrical conduction via the electrolyte in the pore; and then
applying a potential difference between a measuring particle injecting side electrode in the measuring particle injecting side flow path and a measuring particle non-injecting side electrode in the measuring particle non-injecting side flow path, thereby measuring a transient change in ion current between the measuring particle injecting side electrode and the measuring particle non-injecting side electrode when the measuring particle passes through the pore;
wherein a hydrophilic agent does not adhere to the measuring particle injecting side flow path and to the measuring particle injecting side connecting portion.

Another embodiment of the particle measurement method can fill the Si monocrystalline communication portion, the measuring particle non-injecting side flow path connecting portion and the measuring particle non-injecting side flow path with the measuring particle-free electrolyte containing a hydrophilic agent, and/or apply the hydrophilic agent to at least a part of their surfaces, such that the hydrophilic agent adheres to the surfaces, or
such that the measuring particle injecting side connecting portion is at least inclined to the measuring particle non-injecting side flow path.

The particle measurement method can form the particle measuring sensor device such that:
the measuring particle injecting side flow path is formed by being surrounded by: a measuring particle injecting side flow path base surface member in which the measuring particle injecting side flow path connecting portion is opened; a measuring particle injecting side flow path side surface member provided with the measuring particle injecting side flow path; and a measuring particle injecting side flow path cover surface member in which the measuring particle injecting side flow path introduction portion is opened; and
the measuring particle non-injecting side flow path is formed by being surrounded by: a measuring particle non-injecting side flow path base surface member in which the measuring particle non-injecting side flow path connecting portion is opened; a measuring particle non-injecting side flow path side surface member provided with the measuring particle non-injecting side flow path; and a measuring particle non-injecting side flow path cover surface member in which a measuring particle non-injecting side flow path introduction portion is opened.

The particle measurement method can form the particle measuring sensor device such that:
the Si monocrystalline communication portion is provided so that the thin film is in contact with the Si monocrystalline substrate formed on a (100) plane of the Si monocrystal;
the Si monocrystalline communication portion is provided to communicate the measuring particle injecting side flow path with the measuring particle non-injecting side flow path via the pore of the thin film, the pore being provided by penetrating the Si monocrystalline substrate to be applied to pointed tips of inclined surface portions formed in (111) planes of the Si monocrystal so as to be surrounded by the inclined surface portions and the thin film; and
the smallest angle of angles formed by any of tangent lines between base end opening surfaces of the inclined surface portions of the Si monocrystal and the inclined surface portions, and a long side of the measuring particle non-injecting side flow path is 20 degrees or less.

In the particle measurement method, it is preferable to form the particle measurement sensor device such that the angle is 10 degrees or less.

The particle measurement method can form the particle measuring sensor device such that:
a shape of a part or the whole of a portion of the measuring particle non-injecting side flow path connecting portion that is closer to the measuring particle non-injecting side flow path introduction portion than the pore along a longitudinal direction of the measuring particle non-injecting side flow path has a smaller width in a direction orthogonal to the longitudinal direction of the measuring particle non-injecting side flow path as it is closer to the measuring particle non-injecting side flow path introduction portion.

In an embodiment, a particle measuring sensor device according to the present invention made to achieve the above object is a particle measurement sensor device for measuring particles, the particle measurement sensor device having a Si monocrystalline substrate in which a Si monocrystalline communication portion is recessed and a hole to the Si monocrystalline communication portion is opened, wherein a thin film covering the entire hole is in contact with the Si monocrystalline substrate, and the thin film is provided with a pore that is in communication with the hole, and the particle measurement sensor device comprises: a measuring particle injecting side flow path and a measuring particle non-injection side flow path that sandwich the Si monocrystalline substrate and are in communication with the Si monocrystalline communication portion; and a measuring particle injection side flow path connecting portion and a measuring particle non-injection side flow path connecting portion to the Si monocrystalline communication portion; and the particle measurement sensor device is for measuring the particles by filling the measuring particle injecting side flow path and the measuring particle injecting side flow path connecting portion with an electrolyte mixed with measuring particles, and filling the Si monocrystalline communication portion, the measuring particle non-injecting side connecting portion and the measuring particle non-injecting side flow path with a measuring particle-free electrolyte;
wherein a hydrophilic agent does not adhere to the measuring particle injecting side flow path and to the measuring particle injecting side connecting portion;
the Si monocrystalline communication portion, the measuring particle non-injecting side flow path connecting portion and the measuring particle non-injecting side flow path are filled with the measuring particle-free electrolyte containing a hydrophilic agent, and/or the hydrophilic agent is applied to at least a part of their surfaces, whereby the hydrophilic agent adheres to the part of the surfaces; or
the measuring particle injecting side connecting portion is at least inclined to the measuring particle non-injecting side flow path.

In another embodiment, in the particle measuring sensor device,
the thin film is in contact with the Si monocrystalline substrate formed on a (100) plane of a Si monocrystal;
the Si monocrystalline communication portion is formed to communicate the measuring particle injecting side flow path with the measuring particle non-injecting side flow path via the pore of the thin film, the pore being provided by penetrating the Si monocrystalline substrate to be applied to pointed tips of inclined surface portions formed in (111) planes of the Si monocrystal so as to be surrounded by the inclined surface portions and the thin film; and
the smallest angle of angles formed by any of tangent lines between base end opening surfaces of the inclined surface portions of the Si monocrystal and the inclined surface portions, and a long side of the measuring particle non-injecting side flow path is 20 degrees or less.

In the particle measurement sensor device, the angle is more preferably 10 degrees or less.

In the particle measurement sensor device, a shape of a part or the whole of a portion of the measuring particle non-injecting side flow path connecting portion that is closer to the measuring particle non-injecting side flow path introduction portion than the pore along a longitudinal direction of the measuring particle non-injecting side flow path can have a smaller width in a direction orthogonal to the longitudinal direction of the measuring particle non-injecting side flow path as it is closer to the measuring particle non-injecting side flow path introduction portion.

### [Advantageous Effects of Invention]

According to the particle measurement method and the particle measurement sensor device used for the same according to the present invention, air bubbles are difficult to remain in the flow path or the Si monocrystalline substrate (pore chip) by imparting hydrophilicity using a specific hydrophilizing agent and/or by preventing a liquid flow from remaining by a specific structure. Therefore, the test liquid is not blocked by the air bubbles and can be smoothly delivered. Moreover, due to the smooth delivery of the liquid, the risks of causing the clogging with the identifying particles in the test liquid and causing blocking or pulsating flow are eliminated. They allow the particles such as fine particles, viruses and bacteria to be accurately and stably measured, thereby enabling rapid and easy measurement of particles in an increased number of specimens industrially, clinically, and commercially. Therefore, they are applicable to various fields.

### [Brief Description of Drawings]

[FIG. 1]

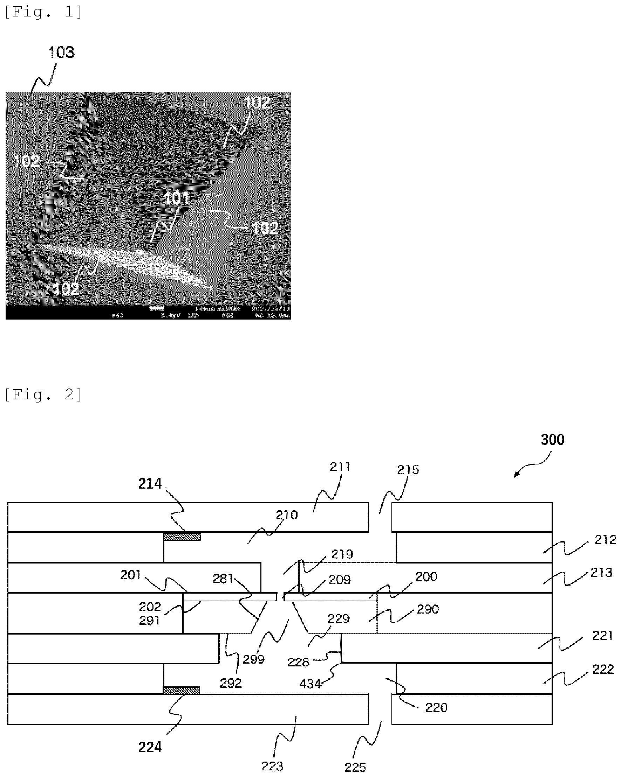

   FIG. 1 is a micrograph showing an outline of a Si monocrystalline substrate in a particle measurement sensor device used for a particle measurement method to which the present invention is applied.
[FIG. 2]
   FIG. 2 is a cross-sectional view of a particle measuring sensor device used for a particle measurement method to which the present invention is applied.
[FIG. 3]

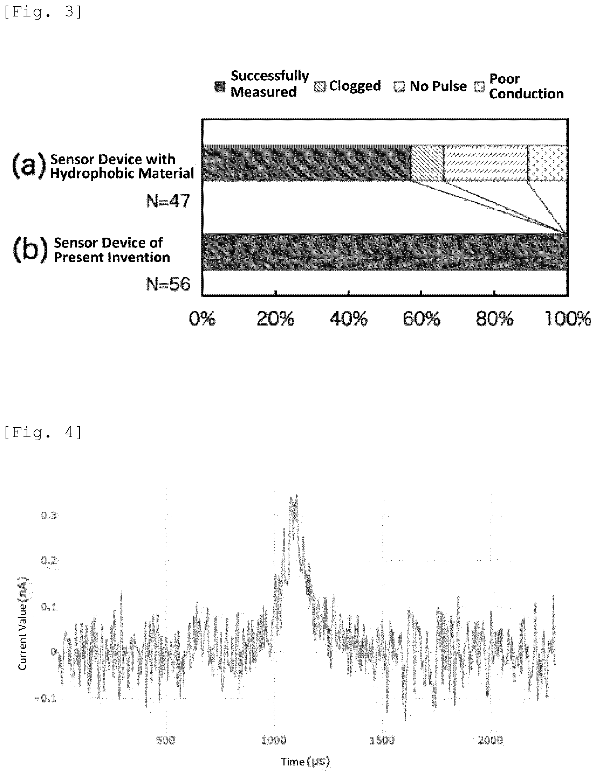

   FIG. 3 is a graph showing measurement results when particles are measured using a particle measurement sensor device using only a hydrophobic material to which the present invention is not applied, and a particle measurement sensor device using a hydrophilizing agent in a particle measurement method to which the present invention is applied.
[FIG. 4]
   FIG. 4 is a chart showing results of particle measurement by a particle measurement sensor device used for a particle measurement method to which the present invention is applied.
[FIG. 5]

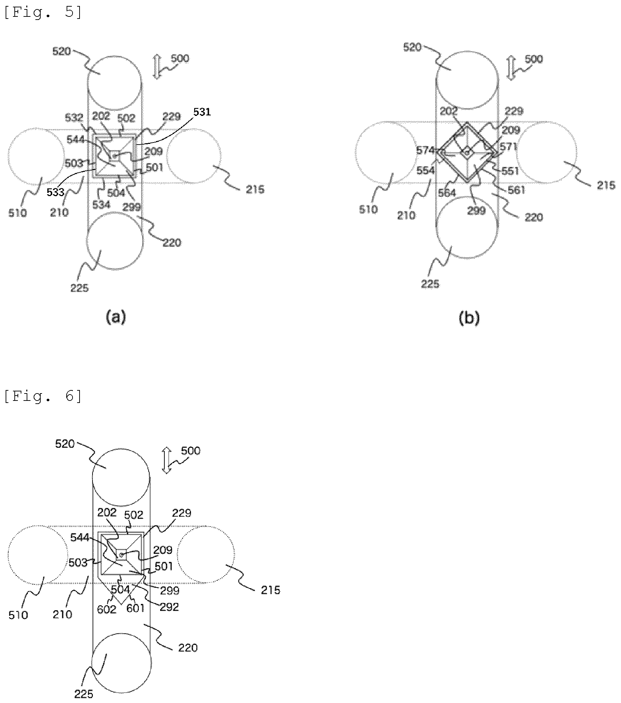

   FIG. 5 is an enlarged schematic view of each of a particle measuring sensor device according to another embodiment of the present invention used for a particle measurement method to which the present invention is applied, and a particle measuring sensor device to which the present invention is not applied.
[FIG. 6]
   FIG. 6 is an enlarged schematic view of a particle measuring sensor device according to still another embodiment used for a particle measurement method to which the present invention is applied.
[FIG. 7]

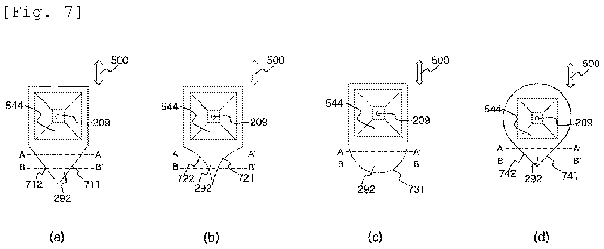

   FIG. 7 is an enlarged schematic view of a particle measuring sensor device according to still another embodiment used for a particle measurement method to which the present invention is applied.

### [Description of Embodiments]

Hereinafter, embodiments for carrying out the present invention will be described in detail, but the scope of the present invention is not limited to these embodiments.

An embodiment of the particle measurement method and a particle measurement sensor device 300 used for the same according to the present invention will be described with reference to schematic views of FIGS. 1 and 2. The particle measurement method uses the particle measurement sensor device 300 having: a Si monocrystalline substrate 290 that is a pore chip made of a Si monocrystal formed from the Si monocrystalline substrate; and a first flow path 210 and a second flow path 220 formed to sandwich the Si monocrystalline substrate from above and below.

To the Si monocrystalline substrate 290 is applied a thin film 200 in which a pyramidal Si monocrystalline communication portion 299 is recessed, and the thin film 200 covers a pointed tip opening 101 of the pyramidal pointed tip.

In the Si monocrystalline substrate 290, the Si monocrystalline communication portion 299 is provided to be recessed into a pyramid shape by anisotropic etching. A second surface (a thin film second surface 202) of the thin film 200 covering the entire pointed tip opening 101 is in contact with a first surface 291 of the Si monocrystalline substrate 290 in which the pointed tip opening 101 of the Si monocrystalline communication portion 299 is opened. A pore 209 that is in communication with the pointed tip opening 101 is formed in the thin film second surface 202. It has a first flow path connecting portion 219 and a second flow path connecting portion 229 that sandwich the Si monocrystalline substrate 290 and are in communication with the Si monocrystalline communication portion 299.

Here, the embodiments of FIGS. 1 and 2 are an example in which the first flow path 210 is on a measuring particle injecting side, and the second flow path 220 is on a measuring particle non-injecting side.

The most important feature of the present invention in such an embodiment consists in measuring particles by filling the first flow path 210 and the first flow path connecting portion 219 of the particle measurement method and the particle measurement sensor device used for the same with an electrolyte that is mixed with measuring particles and does not contain any hydrophilizing agent, filling the Si monocrystalline communication portion 299, the second flow path connecting portion and the second flow path with an electrolyte that is mixed with a hydrophilizing agent for increasing hydrophilicity of their surfaces and does not contain the measuring particles, and bringing the first flow path 210 and the second flow path 220 between a first electrode 214 in the first flow path 210 and a second electrode 224 in the second flow path 220 to a state capable of electrical conduction via the electrolyte in the pore 209; and then applying a potential difference between the first electrode 214 and the second electrode 224, thereby measuring a transient change in ion current between the first electrode 214 and the second electrode 224 when the measuring particles pass through the pore, wherein the hydrophilizing agent is not mixed in the first flow path. This point will be described below in more detail.

FIG. 1 is a micrograph of the Si monocrystalline substrate 290 configuring the particle measuring sensor device 300.

According to the example of FIG. 1, the Si monocrystalline substrate 290 that is a Si monocrystalline chip has the Si monocrystalline communication portion 299 that is recessed in the form of the pyramid, which is formed, using a Si monocrystalline wafer made of a Si monocrystal, by anisotropic etching which selectively etches (111) planes that are four inclined surfaces 102, from a (100) plane 103 on a side opposite to the thin film 200 side of the Si monocrystalline substrate 290 to the inside.

Therefore, the Si monocrystalline communication portion 299 is a space surrounded by the thin film 200 and the inclined surfaces 102. In order to achieve the measurement of the change in electrical resistance caused by the measuring particles to pass through the pore 209 using the particle measurement sensor device 300, the Si monocrystalline communication portion 299 is a space for communicating the measuring particle with the opposite side of the Si monocrystalline communication portion 299 from the Si monocrystalline communication portion 299 through the pore 209 of the thin film 200 attached to the Si monocrystalline substrate 290.

It should be noted that the expressions such as (100) and (111) are Miller indices.

The thin film 200 has the pore 209. The pore 209 is formed near the center of the thin film 200 at the pyramidal pointed tip opening 101 of the Si monocrystalline communication portion 299. The pore diameter of the pore 209 is an average diameter of 10 um or less, and can be optionally set as long as it is larger than the measuring particle so that the measuring particles can pass therethrough. The thin film 200 may be made of a material such as SiNₓ and SiO₂. The pore 209 is much smaller than the Si monocrystalline communication portion 299 and is smaller than the resolution of the photograph, so it is not shown in FIG. 1.

FIG. 2 is a schematic cross-sectional view of one embodiment of the particle measuring sensor device 300.

The thin film 200 is formed on a first surface 291 of the Si monocrystalline substrate 290. The pore 209 is provided in the thin film 200. The pore 209 is in communication with a second surface 292 of the Si monocrystalline substrate 290 via the Si monocrystalline communication portion 299 surrounded by inclined surface portions 281 (corresponding to the inclined surfaces 102 in FIG. 1) and the thin film second surface 202.

The first flow path 210 is formed by being surrounded by: a first flow path lower surface member 213 which is a measuring particle injecting side flow path base surface material in which the first flow path connecting portion 219 is opened; a first flow path side surface member 212 on the measuring particle injecting side provided with the first flow path 210; and a first flow path upper surface member 211 which is a measuring particle injecting side cover surface member in which a first flow path introduction portion 215 is opened. Also, the second flow path 220 is formed by being surrounded by: a second flow path upper surface member 221 which is a measuring particle non-injecting side flow path base surface member in which a second flow path connecting portion 229 is opened; a second flow path side surface member 222 on the measuring particle non-injecting side in which the second flow path 220 is provided; and a second flow path lower surface member 223 which is a measuring particle non-injecting side cover surface member in which a second flow path introduction portion 225 is opened.

The first flow path connecting portion 219 is a through hole opened in the first flow path lower surface member 213, and communicates the first flow path 210 with the pore 209. Also, the second flow path connecting portion 229 is a through hole opened in the second flow path upper surface member 221, and communicates the second flow path 220 with the Si monocrystalline communication portion 299.

FIG. 2 merely illustrates one example of the structure of the particle measuring sensor device 300, and the particle measuring sensor device 300 may be formed by a method such as adhesion of different members of the first flow path upper surface member 211, the first flow path side surface member 212, the first flow path lower surface member 213, the thin film 200, the Si monocrystalline substrate 290, the second flow path upper surface member 221, the second flow path side surface member 222 and the second flow path lower surface member 223, or may be integrally formed of a part or the whole of those members by a method such as injection molding.

It should be noted that, in FIGS. 1 and 2, the upper and lower surfaces are described for convenience, but these do not represent absolute upper and lower positions in this specification, but they represent relative opposing positions. Therefore, the particle measurement sensor device 300 may be configured such that the first flow path upper surface member 211 and the first flow path lower surface member 213 are reversed to the second flow path upper surface member 221 and the second flow path lower surface member 223.

Specifically, in FIG. 2, the members of the first flow path upper surface member 211, the first flow path side surface member 212, the first flow path lower surface member 213 and the thin film first surface 201, and the members of the thin film second surface 202, the second flow path upper surface member 221, the second flow path side surface member 222 and the second flow path lower surface member 223 may be upside down. For example, the second flow path 220 may be on the upper side of the Si monocrystal, the second flow path connecting portion 229 and the Si monocrystalline communication portion 299 may be on the upper side of the thin film 200, which may be connected to the second flow path 220, and further, the first flow path 210 may be on the lower side of the Si monocrystal, and the first flow path connecting portion 219 and the first flow path 210 may be on the lower side of the thin film 200, which may be connected to the first flow path 210.

Further, while the example has been shown in which the first flow path 210 is filled with the electrolyte containing the measuring particles but not containing any hydrophilizing agent, and the second flow path 220 is filled with the electrolyte containing the hydrophilizing agent but not containing the measuring particles, the first flow path 210 may be filled with an electrolyte containing the hydrophilizing agent, and the second flow path 220 may be filled with the electrolyte containing the measuring particles, regardless of the positional relationship of the first flow path 210 and the second flow path 220 with respect to the Si monocrystal.

The electrolyte solution containing no hydrophilizing agent, or preferably the electrolyte solution containing the hydrophilizing agent or the electrolyte solution containing the measuring particles (hereinafter, these will be collectively referred to as an electrolyte or the like) is injected into the first flow path 210 through the first flow path introduction portion 215, and into the second flow path 220 through the second flow path introduction portion 225, respectively. Then, the first electrode 214 is electrically connected to the pore 209 via the electrolyte or the like filled in the first flow path 210, the first flow path connecting portion 219 and the pore 209. Similarly, the second electrode 224 is electrically connected to the pore 209 via the electrolyte or the like filled in the second flow path 220, the second flow path connecting portion 229, and the Si monocrystalline communication portion 299. In this way, the first electrode 214 and the second electrode 224 are electrically connected to each other via the pore 209.

The sensor device having such a structure does not yet have the hydrophilizing agent, which is the most important feature of the present invention, and is disclosed in, for example, Patent Literature 8. However, in this state, to apply the sensor device to industrial products for measuring fine particles or to inspection equipment products for viruses, bacteria and the like, it was necessary to solve the problem of stability of operation. For example, even if an electrolyte or an electrolyte containing the measuring particles was injected through the second flow path introduction portion 225 via a pipette or the like, it remarkably caused the problems that the electrolyte solution was stopped in the middle of the flow path without filling the second flow path 220, or the electrolyte solution filled the flow path but did not fill the second flow path connecting portion 229 and/or the Si monocrystalline communication portion 299, leaving air behind. Similarly, when the electrolyte or the like was injected through the first flow path introduction portion 215, the electrolyte was often stopped in the middle of the flow path without filling the second flow path 220, or although the electrolyte filled the flow path, the electrolyte often did not fill the first flow path connecting portion 219, leaving air behind. It would be due to the fact that the surface that will be in contact with the electrolyte in each of the first flow path upper surface member 211, the first flow path side surface member 212, the first flow path lower surface member 213, the thin film 200, the Si monocrystalline substrate 290, the second flow path upper surface member 221, the second flow path side surface member 222 and the second flow path lower surface member 223 are hydrophobic.

Furthermore, due to air bubbles remaining in the second flow path connecting portion 229, the Si monocrystalline communication portion 299 or the first flow path connecting portion 219, a phenomenon often occurs in which the electrical resistance between the first electrode 214 and the second electrode 224 is increased or unstable over time, compared to the case where the electrolyte or the like is filled without air bubbles, or particle clogging occurs, making it difficult to observe particle passing pulses. This is also considered to be due to the same cause.

When such a conventional particle measurement sensor device 300 was used, which was formed of a Si monocrystalline substrate 290 made of Si and was a hydrophobic material with an untreated surface, a thin film 200, a first flow path upper surface member 211, a first flow path side surface member 212, a first flow path lower surface member 213, a second flow path upper surface member 221, a second flow path side surface member 222, and a second flow path lower surface member 223, the pulse measurement success rate was only 60%, and reliability as a particle measurement means could not be ensured, as shown in FIG. 3(a) showing the breakdown of the pulse measurement success rate and the causes of failure as in the case where saliva containing the measuring particles was used as the test liquid.

In order to achieve stable measurement, an approach is considered, which mixes the hydrophilizing agent that will increase the hydrophilicity of the interface making up the flow path into the electrolyte, particularly the measuring particle-containing electrolyte, thereby promoting communication and electrical conduction between the two flow paths through the pore. However, when the measuring particles are so-called bioparticles such as viruses, bacteria and extracellular vesicles, the hydrophilizing agent that will increase the hydrophilicity of the interface often destroys those bioparticles or at least affects their morphology and properties. Therefore, such an approach is not suitable for applications such as measurement of the number of bioparticles or estimation of some properties of the measuring bioparticles from the transient change in the current between the first electrode 214 and the second electrode 224.

To solve such problems, the present invention is configured to have the hydrophilizing agent, which is the most important feature as described above. According to a preferred embodiment of the present invention, the problem of the second flow path 220 and the second flow path connecting portion 229 and the Si monocrystalline communication portion 299 which have more complicated shapes than those of the first flow path 210 and the first flow path connecting portion 219 and is therefore likely to have air bubbles remaining therein, is solved by the following configuration.

Specifically, first, the electrolyte containing the measuring particles is injected through the first flow path introduction portion 215 to fill the first flow path 210, the first flow path connecting portion 219 and the pore 209. Also, the electrolyte mixed with the hydrophilizing agent that will increase the hydrophilicity of the interface making up the second flow path 220 is injected through the second flow path 220 to fill the second flow path 220, the second flow path connecting portion 229, the Si monocrystalline communication portion 299, and the pore 209. Such a configuration allows for more stable electrical conduction between the first electrode 214 and the second electrode 224.

In this state, for example, the application of the positive voltage to the second electrode 224 relative to the first electrode 214 allows the negatively charged measuring particles in the first flow path 210 to be moved by electrophoresis through the first flow path connecting portion 219 and the pore 209 to the Si monocrystalline communication portion 299. In this way, when the measuring particles pass through the pore 209, they temporarily block the ion current flowing through the pore 209, resulting in a transient change in the current flowing between the first electrode 214 and the second electrode 224. This is a signal due to the particles passing through the pore, and this can be detected and analyzed to derive the number and type of measuring particles. Similarly, the application of the negative voltage to the second electrode 224 relative to the first electrode 214 allows the positively charged measuring particles in the first flow path 210 to be moved by electrophoresis through the first flow path connecting portion 219 and the pore 209 to the Si monocrystalline communication portion 299. In this case, the transient change in the current flowing between the first electrode 214 and the second electrode 224 can also be measured and analyzed.

In this case, the electrolyte and the measurement target particles are present in the second flow path 220, but no hydrophilizing agent is present. Therefore, the measuring biological particles in the first flow path 210 are not affected by the hydrophilizing agent until they have completely passed through the pore 209. This allows stable particle measurement to be carried out while eliminating the effect of the hydrophilizing agent on the measuring particles.

For example, FIG. 4 shows an example in which, using an influenza A (H1N1) virus culture supernatant, the electrolyte containing influenza viruses, which are the measuring particles, is introduced into the first flow path 210 and the electrolyte containing the hydrophilizing agent is introduced into the second flow path 220, thereby measuring a pulse signal based on the passage of the particles through the pore. In FIG. 4, one pulse is a signal based on the passage of one virus particle through the pore. Thus, this shows that the measurement target in the first flow path is not affected by the hydrophilizing agent.

As the hydrophilizing agent, surfactants can be used. Among them, a silicone-based surfactant is preferable, and a silicone linear polyether-modified silicone is more preferable.

Although an example has been shown in which the hydrophilizing agent is contained in the test liquid such as the electrolyte and injected into the second flow path 220 as the particle measurement sensor device 300, any embodiment may be used as long as no hydrophilizing agent adheres to the measuring particle injecting side flow path and the measuring particle injecting side flow path connecting portion. For example, the hydrophilizing agent or the like that will increase the hydrophilicity may be applied to the flow path side inner surface of at least one of any members among the first flow path upper surface member 211, the first flow path side surface member 212, the first flow path lower surface member 213, the thin film first surface 201, the thin film second surface 202, the second flow path upper surface member 221, the second flow path side surface member 222, and the second flow path lower surface member 223. Alternatively, as the particle measurement sensor device 300, the hydrophilizing agent that will increase the hydrophilicity of the member surface may be mixed into any one or more of the first flow path upper surface member 211, the first flow path side surface member 212, the first flow path lower surface member 213, the thin film first surface 201, the thin film second surface 202, the second flow path upper surface member 221, the second flow path side surface member 222 and the second flow path lower surface member 223.

Even in the case of the particle measurement sensor device 300 using the Si monocrystalline substrate 290, which is the Si monocrystalline chip having the thin film 200 and the pore 209 as shown in FIG. 2, in an embodiment where the hydrophilizing agent is not used as in the prior art, the electrolyte does not enter the Si monocrystalline communication portion 299, and the electrical conduction between the electrodes is often failed. Alternatively, although the electrolyte enters the Si monocrystalline communication portion 299, the air bubbles remain in a part of it, which frequently causes problems that no electrical conduction occurs between the electrodes, or it results in a higher resistance value than that in the case of proper electrical conduction. However, by using the hydrophilizing agent as in the present invention, these problems are solved. Therefore, it is effective to fill the flow path on the Si monocrystalline communication portion side with the electrolyte containing the hydrophilizing agent, and fill the flow path on the opposite side with the electrolyte containing the measuring particles.

Next, another embodiment of the particle measuring sensor device 300 to which the present invention is applied will be described.

The conventional particle measurement sensor device 300 has a structure in which the pore 209 formed in the thin film 200 and the opposite surface of the Si monocrystalline substrate to the thin film 200 are in communication with each other by anisotropic etching of the (111) planes, so that the Si monocrystalline communication portion 299 is surrounded by the four (111) planes. Therefore, the tangent line between the pointed tip opening 101 and the inclined surfaces 102 is a square as shown in FIG. 1. Similarly, the tangent line between the inclined surface 102 and the (100) surface 103 opposite to the pointed tip opening 101 of the Si monocrystalline substrate 290 is also a square as shown in FIG. 1. Therefore, the Si monocrystalline communication portion 299 is a quadrangular pyramid in which both upper and lower surfaces are square. However, if the hydrophilizing agent is not used, turbulence is generated when the electrolyte or the like flows into the Si monocrystalline communication portion 299 having that shape, resulting in the entrainment of air bubbles, which causes the air bubbles to remain in the Si monocrystalline communication portion 299. The use of the hydrophilizing agent improves compatibility with an aqueous medium of the electrolyte or the like, facilitates the flowing of the electrolyte or like without generating turbulence, and leads to difficulty to entrain the air bubbles.

FIG. 5B which is first described is an example of the structure of the conventional particle measuring sensor device 300. FIG. 5(b) is a bottom view of the particle measuring sensor device 300 as viewed from the second flow path lower surface member 223 in the cross-sectional view shown in FIG. 2. The electrolyte or the like is injected into the second flow path 220 through the second flow path introduction portion 225. As a result, the electrolyte or the like first proceeds in a second flow path longitudinal direction 500 from the second flow path introduction portion 225 to an air vent hole 520. Then, the electrolyte or the like flows into the second flow path connecting portion 229 beyond edges 561 and 564 of the second flow path communication portion 229 formed in a 45-degree direction with respect to the second flow path longitudinal direction 500. Further, the electrolyte or the like flows into the Si monocrystalline communication portion 299 beyond edges 551 and 554 of the Si monocrystalline communication portion 299 formed in a 45-degree direction with respect to the second flow path longitudinal direction 500. At this time, the electrolyte or the like flows in from an oblique direction toward the thin film second surface 202 along both an inclined surface 571 side and an inclined surface 574 side, and these two flows collide with each other near the thin film second surface 202. Therefore, there has been problems that the air near the center of the Si monocrystalline communication portion 299 is easily entrained, and even after the electrolyte or the like passes through the Si monocrystalline communication portion 299 and reaches the air vent hole 520, the air bubbles easily remain in the Si monocrystalline communication portion 299.

In contrast, FIG. 5(a) is a part of the structure according to another embodiment to which the present invention is applied. Fig. 5(a) is a bottom view of the conventional particle measurement sensor device 300 as viewed from the direction of the second flow path lower surface member 223 in the cross-sectional view shown in FIG. 2. In the example of FIG. 5, the longitudinal directions of the first flow path 210 and the second flow path 220 (second flow path longitudinal direction 500) are orthogonal to each other. Reference numerals 510 and 520 are air vent holes of the first flow path 210 and the second flow path 220, respectively.

According to the particle measuring sensor device 300 to which the present invention is applied, like FIG. 5 (a), the electrolyte or the like is injected into the second flow path 220 through the second flow path introduction portion 225. As a result, the electrolyte or the like first proceeds from the second flow path introduction 225 through the second flow path 220 in the second flow path longitudinal direction 500 toward the air vent hole 520. Then, it flows into the second flow path connecting portion 229 beyond the edge 534 of the second flow path connecting portion 229, and further flows into the Si monocrystalline communication portion 299 beyond the edge 504 of the Si monocrystalline communication portion 299 formed in the direction perpendicular to the second flow path longitudinal direction 500. After that, when the pore 209 and the Si monocrystalline communication portion 299 are filled, the electrolyte further proceeds beyond the edge 502 of the Si monocrystalline communication portion 299 and the edge 532 of the second flow path connecting portion 229 toward the air vent hole 520. In the present invention, the electrolyte reaches the thin film second surface 202 without generating turbulence along the inclined surface 544 after passing over the edge 504 of the Si monocrystalline communication portion 299. Therefore, the Si monocrystalline communication portion 299 can be filled with the electrolyte or the like without entraining the air bubbles.

In the example shown in FIG. 5(a), the edges 531 to 534 of the second flow path connecting portion 229 are square like the edges 501 to 504 of the Si monocrystalline communication portion 299, but the edges 531 to 534 of the second flow path connecting portion 229 may have any shape. In the present invention, if the smallest angle among the angles formed by each of the four edges 501, 502, 503, and 504 of the Si monocrystalline communication portion 299 formed into the square and the second flow path longitudinal direction 500 is 20 degrees or less, the turbulence caused by the electrolyte or the like flowing in from an oblique direction toward the thin film second surface 202 and colliding with each other can be prevented. The smaller the smallest angle is, the greater the effect of preventing the turbulence, but in view of manufacturing variations, it is appropriate to set it to 20 degrees or less.

In the example of FIG. 5, the edge 504 of the Si monocrystalline communication portion 299 is perpendicular or nearly perpendicular to the second flow path longitudinal direction 500, thereby obtaining the effect of preventing the turbulence in the Si monocrystalline communication portion 299.

Next, still another embodiment of the particle measuring sensor device 300 to which the present invention is applied will be described.

First, referring to FIG. 2 again, if the hydrophilizing agent is not used, the step from the second flow path 220 to the second flow path connecting portion 229 becomes larger when the thickness of the second flow path upper surface material 221 is larger, and as a result, the turbulence may occur when the electrolyte passes through that step. In this case, there are problems that the air bubbles also remain in the second flow path connecting portion 229 or the Si monocrystalline communication portion 299, and the electrical conduction between the first electrode 214 and the second electrode 224 becomes poor.

Using such a conventional particle measuring sensor device 300, the electrolyte or the like is injected into the second flow path 220 through the second flow path introduction portion 225. As a result, the electrolyte or the like first proceeds through the second flow path 220 from the second flow path introduction portion 225 in the second flow path longitudinal direction 500 to the air vent hole 520. At this time, as already described with FIG. 5 (b), the electrolyte flows in from the inclined surfaces of the two directions at the Si monocrystalline communication portion 299 and collides with each other, which causes the air bubbles. That is, in the flow path formed by the inclined surface portion 281 in FIG. 2, the movement of the fluid in the lateral direction to the second flow path longitudinal direction 500 causes the air bubbles. Also, the edges 531 to 534 of the second flow path connecting portion 229 in FIG. 2 and FIG. 5 (a) are formed by a vertical surface 228 perpendicular to the flow path longitudinal direction, rather than the inclined surface. In this case, the electrolyte or the like that has passed through the edge 534 of the second flow path connecting portion 229 falls down the step in the direction perpendicular to the second flow path longitudinal direction 500. Therefore, if the second flow path upper surface member 221 is thicker, there is a possibility that the problem of the air bubbles being entrained here may occur.

FIG. 6 shows an example of a structure according to yet another embodiment of the particle measuring sensor device 300 according to the present invention for solving that problem.

As shown in FIG. 6, when the electrolyte or the like is injected into the second flow path 220 through the second flow path introduction portion 225, the electrolyte or the like first proceeds through the second flow path 220 from the second flow path introduction portion 225 in the second flow path longitudinal direction 500 to the air vent hole 520. The electrolyte solution then passes over the edges 601 and 602 of the second flow path connecting portion 229 and sequentially flows into the second surface 292 of the Si monocrystalline substrate 290. In an example of FIG. 6, the edges 601 and 602 of the second flow path connecting portion 229 are disposed in an oblique direction with respect to the flow path longitudinal direction. As a result, the electrolyte or the like gradually reaches the second surface 292 of the Si monocrystalline substrate 290, so that the air bubble entrainment in the second flow path connecting portion 229, which is caused by the vertical surface 228 with respect to the second flow path longitudinal direction 500, can be prevented.

The shape of the second flow path connecting portion 229 composed of the edges 601 and 602 shown in FIG. 6 is illustrative, and is not particularly limited as long as the edge 534 of the edges 531 to 534 of the Si monocrystalline communication portion 299 has a shape such that its width decreases from the side closest to the second flow path introduction portion 225 (504 in an example of FIG. 5 (a)) to the second flow path introduction portion 225.

Specifically, Fig. 7 shows the shapes of the second flow path connecting portions 229 as an example of the present invention. In each of those shown in FIG. 7, the second flow path introduction portion 225 is in the down direction, and the electrolyte or the like proceeds in the up direction along the second flow path longitudinal direction 500. In each of those shown in FIG. 7, the shape of the second flow path connecting portion 229 is structured such that the width becomes narrower as it is closer to the second flow path introduction portion 225 in the second flow path longitudinal direction 500 by sides 711 and 712 in (a), arcs 721 and 722 in (b), arc 731 in (c), and sides 741 and 742 in (d). That is, in (a) to (d) of FIG. 7, the width of the second flow path connecting portion 229 is shorter at B-B' than at A-A' in the second flow path longitudinal direction 500.

FIG. 3 shows comparison results of measurement success rates when measuring the passage of the measuring particles through the pore in the particle measurement sensor device (a) according to the conventional structure and method and the particle measurement sensor device (b) according to the structure and method of the present invention. The particle measurement sensor device according to the structure and method of the present invention was the particle measurement sensor device 300 having the structure of the Si monocrystalline communication portion 299 and the structure of the second flow path connecting portion 229 shown in FIG. 6, and the electrolyte containing the measuring particles was introduced into the first flow path 210, and the electrolyte containing the hydrophilizing agent was introduced into the second flow path 220. As shown in FIG. 3, it was found that the structure and method according to the present invention had a significant effect on stable measurement of the electrical resistance porosity analysis using such a particle measurement sensor device as compared to the conventional one.

### [Industrial Applicability]

The particle measurement method according to the present invention and the particle measurement sensor device used for the same are useful for measuring the physical properties of submicron-sized particles such as viruses, bacteria, exosomes, and suspended particles in a specimen, and in particular for particle measurement based on electrical resistance porosity analysis, particle coefficient, and/or particle identification.

### [Description of Reference Numerals]

101 pointed tip opening
102 inclined surface
103 surface
200 thin film
201 thin film first surface
202 thin film second surface
209 pore
210 first flow path (measuring particle injecting side flow path)
211 first flow path upper surface member
212 first flow path side surface member
213 first flow path lower surface member
214 first electrode
215 first flow path introduction portion
219 first flow path connecting portion
220 second flow path (measuring particle non-injecting side flow path)
221 second flow path upper surface member
222 second flow path side surface member
223 second flow path lower surface member
224 second electrode,
225 second flow path introduction portion
228 vertical surface
229 second flow path connecting portion
281 inclined portion
290 Si monocrystalline substrate
291 first surface
292 second surface
299 Si monocrystalline communication portion
300 particle measurement sensor device
500 second flow path longitudinal direction
501, 502, 503, 504 edge
510, 520 air vent hole
531, 532, 533, 534 edge
544 inclined portion
551, 554 edge
561, 564 edge
571, 574 inclined portion
601, 602 edge
711, 712 side
731 arc
741, 742 side

## Claims

1. A particle measurement method, the method comprising:
using a particle measurement sensor device having a Si monocrystalline substrate in which a Si monocrystalline communication portion is recessed and a pointed tip opening to the Si monocrystalline communication portion is opened, wherein a thin film covering the entire pointed tip opening is in contact with the Si monocrystalline substrate, and the thin film is provided with a pore that is in communication with the pointed tip opening, and the particle measurement sensor device comprises: a measuring particle injecting side flow path and a measuring particle non-injection side flow path that sandwich the Si monocrystalline substrate and are in communication with the Si monocrystalline communication portion; and a measuring particle injection side flow path connecting portion and a measuring particle non-injection side flow path connecting portion to the Si monocrystalline communication portion; and
filling the measuring particle injecting side flow path and the measuring particle injecting side flow path connecting portion with an electrolyte mixed with measuring particles, filling the Si monocrystalline communication portion, the measuring particle non-injecting side connecting portion and the measuring particle non-injecting side flow path with a measuring particle-free electrolyte, and bringing the measuring particle injecting side flow path and the measuring particle non-injecting side flow path to a state capable of electrical conduction via the electrolyte in the pore; and then
applying a potential difference between a measuring particle injecting side electrode in the measuring particle injecting side flow path and a measuring particle non-injecting side electrode in the measuring particle non-injecting side flow path, thereby measuring a transient change in ion current between the measuring particle injecting side electrode and the measuring particle non-injecting side electrode when the measuring particle passes through the pore;
wherein a hydrophilic agent does not adhere to the measuring particle injecting side flow path and to the measuring particle injecting side connecting portion.

2. The particle measurement method according to claim 1, wherein the hydrophilic agent adheres to the surfaces by filling the Si monocrystalline communication portion, the measuring particle non-injecting side flow path connecting portion and the measuring particle non-injecting side flow path with the measuring particle-free electrolyte containing a hydrophilic agent, and/or applying the hydrophilic agent to at least a part of their surfaces, or
the measuring particle injecting side connecting portion is at least inclined to the measuring particle non-injecting side flow path.

3. The particle measurement method according to claim 1 or 2, wherein the particle measuring sensor device is formed such that:
the measuring particle injecting side flow path is formed by being surrounded by: a measuring particle injecting side flow path base surface member in which the measuring particle injecting side flow path connecting portion is opened; a measuring particle injecting side flow path side surface member provided with the measuring particle injecting side flow path; and a measuring particle injecting side flow path cover surface member in which the measuring particle injecting side flow path introduction portion is opened; and
the measuring particle non-injecting side flow path is formed by being surrounded by: a measuring particle non-injecting side flow path base surface member in which the measuring particle non-injecting side flow path connecting portion is opened; a measuring particle non-injecting side flow path side surface member provided with the measuring particle non-injecting side flow path; and a measuring particle non-injecting side flow path cover surface member in which a measuring particle non-injecting side flow path introduction portion is opened.

4. The particle measurement method according to any one of claims 1 to 3, wherein the particle measuring sensor device is formed such that:
the Si monocrystalline communication portion is provided so that the thin film is in contact with the Si monocrystalline substrate formed on a (100) plane of the Si monocrystal;
the Si monocrystalline communication portion is provided to communicate the measuring particle injecting side flow path with the measuring particle non-injecting side flow path via the pore of the thin film, the pore being provided by penetrating the Si monocrystalline substrate to be applied to pointed tips of inclined surface portions formed in (111) planes of the Si monocrystal so as to be surrounded by the inclined surface portions and the thin film; and
the smallest angle of angles formed by any of tangent lines between base end opening surfaces of the inclined surface portions of the Si monocrystal and the inclined surface portions, and a long side of the measuring particle non-injecting side flow path is 20 degrees or less.

5. The particle measurement method according to claim 4, wherein the particle measurement sensor device is formed such that the angle is 10 degrees or less.

6. The particle measurement method according to any one of claims 1 to 5, wherein the particle measuring sensor device is formed such that:
a shape of a part or the whole of a portion of the measuring particle non-injecting side flow path connecting portion that is closer to the measuring particle non-injecting side flow path introduction portion than the pore along a longitudinal direction of the measuring particle non-injecting side flow path has a smaller width in a direction orthogonal to the longitudinal direction of the measuring particle non-injecting side flow path as it is closer to the measuring particle non-injecting side flow path introduction portion.

7. A particle measurement sensor device, the particle measurement sensor device having a Si monocrystalline substrate in which a Si monocrystalline communication portion is recessed and a hole to the Si monocrystalline communication portion is opened, wherein a thin film covering the entire hole is in contact with the Si monocrystalline substrate, and the thin film is provided with a pore that is in communication with the hole, and the particle measurement sensor device comprises: a measuring particle injecting side flow path and a measuring particle non-injection side flow path that sandwich the Si monocrystalline substrate and are in communication with the Si monocrystalline communication portion; and a measuring particle injection side flow path connecting portion and a measuring particle non-injection side flow path connecting portion to the Si monocrystalline communication portion; and the particle measurement sensor device is for measuring particles by filling the measuring particle injecting side flow path and the measuring particle injecting side flow path connecting portion with an electrolyte mixed with measuring particles, and filling the Si monocrystalline communication portion, the measuring particle non-injecting side connecting portion and the measuring particle non-injecting side flow path with a measuring particle-free electrolyte;
wherein a hydrophilic agent does not adhere to the measuring particle injecting side flow path and to the measuring particle injecting side connecting portion;
wherein the Si monocrystalline communication portion, the measuring particle non-injecting side flow path connecting portion and the measuring particle non-injecting side flow path are filled with the measuring particle-free electrolyte containing a hydrophilic agent, and/or the hydrophilic agent is applied to at least a part of their surfaces, whereby the hydrophilic agent adheres to the part of the surfaces; or
wherein the measuring particle injecting side connecting portion is at least inclined to the measuring particle non-injecting side flow path.

8. The particle measuring sensor device according to claim 7, wherein:
the thin film is in contact with the Si monocrystalline substrate formed on a (100) plane of a Si monocrystal;
the Si monocrystalline communication portion is formed to communicate the measuring particle injecting side flow path with the measuring particle non-injecting side flow path via the pore of the thin film, the pore being provided by penetrating the Si monocrystalline substrate to be applied to pointed tips of inclined surface portions formed in (111) planes of the Si monocrystal so as to be surrounded by the inclined surface portions and the thin film; and
the smallest angle of angles formed by any of tangent lines between base end opening surfaces of the inclined surface portions of the Si monocrystal and the inclined surface portions, and a long side of the measuring particle non-injecting side flow path is 20 degrees or less.

9. The particle measurement sensor device according to claim 8, wherein the angle is 10 degrees or less.

10. The particle measurement sensor device according to any one of claims 7 to 9, wherein a shape of a part or the whole of a portion of the measuring particle non-injecting side flow path connecting portion that is closer to the measuring particle non-injecting side flow path introduction portion than the pore along a longitudinal direction of the measuring particle non-injecting side flow path has a smaller width in a direction orthogonal to the longitudinal direction of the measuring particle non-injecting side flow path as it is closer to the measuring particle non-injecting side flow path introduction portion.
